# EUROPÄISCHE PATENTSCHRIFT

(11) **EP 1 258 485 B1**
(45) Veröffentlichungstag und Bekanntmachung des Hinweises auf die Patenterteilung: **22.06.2005**
(21) Anmeldenummer: 02010129.1
(22) Anmeldetag: 10.05.2002
(51) Int. Cl.: C07D 487/08

(54) **Aufarbeitung der bei der Herstellung von hochreinem Triethylendiamin anfallenden Mutterlauge**
Method for recycling of mother liquor from a high-purity triethylenediamine production process
Procédé de recyclage de la solution-mère issue du processus de production de la triéthylènediamine de haute pureté

(30) Priorität: 10.05.2001 DE 10122502
(43) Veröffentlichungstag der Anmeldung: 20.11.2002
(73) Patentinhaber: BASF AKTIENGESELLSCHAFT, 67056 Ludwigshafen (DE)
(72) Erfinder: Lang, Ortmund, 66909 Quirnbach (DE); Rumpf, Bernd, Dr., 68766 Hockenheim (DE); Frauenkron, Matthias, Dr., 67251 Freinsheim (DE); Funhoff, Dirk, Dr., 68165 Mannheim (DE); Manderbach, Thomas, Dr., 67063 Ludwigshafen (DE); Stein, Bernd, Dr., 64665 Alsbach-Hähnlein (DE)
(74) Vertreter: Isenbruck, Günter, Dr.

(56) Entgegenhaltungen:
- EP-A- 1 070 717
- US-A- 3 400 129
- US-A- 4 233 447

## Beschreibung

Die vorliegende Erfindung betrifft ein Verfahren zur Aufarbeitung von Mutterlauge, die bei der Herstellung von Triethylendiamin (TEDA) anfällt, das einer bestimmten Aufreinigung unterzogen wurde. Diese Aufreinigung besteht darin, TEDA zu verdampfen, dieses in gasförmiger Form in ein flüssiges Lösungsmittel einzuleiten und das TEDA aus der entstandenen Lösung auszukristallisieren. Die danach erhaltene Mutterlauge wird dann erfindungsgemäß aufgearbeitet.

TEDA ist ein bedeutender Katalysator für die Herstellung von Polyurethanschäumen. TEDA liegt bei Raumtemperatur als Feststoff vor. Zu seiner Herstellung und auch Reinigung sind verschiedene Verfahren bekannt, u.a. diejenigen, die in den nachfolgend genannten Druckschriften offenbart sind:

DE-A 24 42 929; US 3,297,701; DE-A 36 34 258; DE-A 17 45 627; DE-A 37 18 395; EP-A 111 928; EP-A 382 055; EP-A 842 935, EP-A 842 936; EP-A 831 096; EP-A 952 152 und US 5,741,906.

Die bisher bekannten Verfahren der Herstellung von TEDA führen zu Bildung von Produktgemischen, die neben TEDA noch Wasser, Nebenprodukte wie Piperazin und hochmolekulare Verbindungen sowie das gegebenenfalls bei der Umsetzung eingesetzte Lösungsmittel enthalten. TEDA wird aus diesen Gemischen gewöhnlich durch diskontinuierliche oder kontinuierliche Destillation oder Rektifikation abgetrennt und meist in einem anschließenden Schritt durch Kristallisieren oder Umkristallisieren gereinigt.

TEDA kann ohne Eintreten von Qualitätsverschlechterung, insbesondere der Farbe und Farbstabilität, des Geruchs und der Reinheit, lediglich unter vergleichsweise hohem Aufwand gehandhabt werden.

Für die bekannten, üblichen Anwendungen wird generell ein sehr reines, geruchsloses und reinweißes TEDA gefordert. Die nachfolgend genannten Anmeldungen offenbaren Verfahren, die eine entsprechende TEDA-Qualität liefern sollen:

DE-A 26 11 069; DE-A 28 49 993 und JP-A 49 048 609.

Nachteilig an diesen Verfahren ist, daß sie das TEDA nicht in der gewünschten Qualität liefern.

Die Patentanmeldungen DE 199 33 850.7 vom 23.07.1999 und DE 199 62 455.0 vom 22.12.1999 der Anmelderin betreffen ein Verfahren zur Herstellung von reinem TEDA, bei denen man TEDA verdampft und das dampfförmige TEDA in ein flüssiges Lösungsmittel einleitet sowie das TEDA aus dieser Lösung auskristallisiert.

Die Anmeldung DE 101 00 943.7 der Anmelderin vom 10.01.2001 beschreibt ein Verfahren zur Herstellung einer Lösung von reinem TEDA, das dadurch gekennzeichnet ist, daß man TEDA aus einer Mischung enthaltend ein Lösungsmittel oder ein Verdünnungsmittel, das bei Normaldruck einen Siedepunkt im Bereich von 175 bis 250°C aufweist, verdampft und das dampfförmige TEDA in ein flüssiges Lösungsmittel einleitet. Durch anschließendes Auskristallisieren des TEDA aus der so erhaltenen Lösung wird reines TEDA hoher Qualität erhalten.

Nach einer Fest-Flüssig-Trennung des kristallinen TEDA von dem Lösungsmittel wird bei den drei letztgenannten Verfahren eine Mutterlauge erhalten, die neben TEDA unerwünschte Neben- und Zersetzungsprodukte enthält. Aufgrund der Verunreinigungen kann die Mutterlauge nicht erneut in dem Verfahren verwendet werden. Bis jetzt war es nicht möglich, diese Mutterlauge derart aufzuarbeiten, daß sie wiederverwertet oder gar das in der Mutterlauge enthaltene TEDA zurückgewonnen werden konnte. Daher wurde die Mutterlauge generell verworfen. Somit waren eine hohe Menge an Lösungsmittel notwendig sowie die Verluste an in der Mutterlauge verbliebenem TEDA unerwünscht hoch.

Die Aufgabe der vorliegenden Erfindung besteht darin, ein Verfahren zur Verfügung zu stellen, das es gestattet, bei dem Verfahren der Gewinnung von TEDA, das ein Verdampfen des als Ausgangsprodukt eingesetzten TEDA, Einleiten des gasförmigen TEDA in ein Lösungsmittel und Auskristallisieren des TEDA aus der Lösung umfaßt, das verwendete Lösungsmittel zumindest teilweise in den Prozeß zurückzuführen und gegebenenfalls die Ausbeute an TEDA zu erhöhen.

Diese Aufgabe wird gelöst durch ein Verfahren zur Reinigung von TEDA, bei dem TEDA verdampft und das dampfförmige TEDA in ein flüssiges Lösungsmittel eingeleitet und anschließend aus diesem auskristallisiert wird, dadurch gekennzeichnet, daß die nach dem Auskristallisieren des TEDA erhaltene Mutterlauge mit einem Extraktionsmittel extrahiert wird, das nicht oder nur gering mit dem Lösungsmittel der Mutterlauge mischbar ist und in dem sich TEDA gut löst, und die nach Extraktion erhaltene, an TEDA verarmte Mutterlauge und/oder das zur Extraktion verwendete, mit TEDA angereicherte Extraktionsmittel in den Prozeß zurückführt.

Der erste Schritt des erfindungsgemäßen Verfahrens umfaßt die Reaktionsschritte des Reinigens von TEDA, die in den Anmeldungen DE 199 33 850.7, 199 62 455.0 und DE 101 00 943.7 der Anmelderin beschrieben sind. Die in den genannten Anmeldungen beschriebenen Verfahren der Reinigung von TEDA sind ein integraler Bestandteil des Verfahrens nach der vorliegenden Anmeldung und durch Bezugnahme in diese eingeschlossen. Die Verfahren werden nachfolgend noch einmal kurz dargestellt.

Durch das Einleiten des dampfförmigen TEDA in ein flüssiges Lösungsmittel (TEDA-Quench) wird die Bildung von unerwünschten Nebenprodukten, die zur Qualitätsminderung führen, entscheidend verringert.

Als Lösungsmittel für diesen TEDA-Quench eignen sich eine Vielzahl organischer Lösungsmittel. Beispiele umfassen aliphatische, cyclische oder acyclische Kohlenwasserstoffe, insbesondere cyclische und acyclische, verzweigte oder unverzweigte Alkane oder Alkangemische, beispielsweise n-Pentan, i-Pentan, Cyclopentan, Hexan, Cyclohexan, Heptan, Octan und Petrolether, chlorierte aliphatische Kohlenwasserstoffe, insbesondere chlorierte Alkane, beispielsweise Dichlormethan, Trichlormethan, Dichlorether und Trichlorether, aromatische Kohlenwasserstoffe, beispielsweise Benzol, Toluol und Xylole, chlorierte aromatische Kohlenwasserstoffe, beispielweise Chlorbenzol, Alkohole, beispielsweise Methanol, Ethanol, Ethylenglykol, 1,4-Butandiol und Polyetheralkohole, insbesondere Polyalkylenglykole, beispielsweise Diethylenglykol, Dipropylenglykol, Monoethylenglykol oder 1,4-Butandiol, Ketone, insbesondere aliphatische Ketone, beispielsweise Aceton, Methylethylketon und Diethylketon, aliphatische Carbonsäureester, beispielsweise Essigsäuremethylester und Essigsäureethylester, aliphatische Nitrile, beispielsweise Acetonitril und Propionitril, Ether, beispielsweise Dioxan, THF, Diethylether und Ethylenglykoldimethylether sowie Gemische der vorstehend aufgeführten Lösungsmittel.

Als Lösungsmittel für den TEDA-Quench wird vorzugsweise ein aliphatischer Kohlenwasserstoff oder ein Polyalkylenglykol, insbesondere ein gesättigter cyclischer oder acyclischer, aliphatischer Kohlenwasserstoff mit 5 bis 8 C-Atomen, beispielsweise Pentan, Hexan, Cyclohexan oder Heptan, oder Dipropylenglykol, verwendet. Die Kristallisation des reinen TEDA aus der erfindungsgemäß hergestellten TEDA-Lösung kann nach dem dem Fachmann bekannten Verfahren erfolgen. Die durch eine nachfolgende mehrstufige, vorzugsweise einstufige Kristallisation erhaltenen TEDA-Kristalle sind hochrein.

Das Einleiten des dampfförmigen TEDA in das flüssige Lösungsmittel erfolgt in einem Quench-Apparat, vorzugsweise einem Fallfilmkondensator (Dünnschicht-, Rieselfilm- oder Fallstromkondensator) oder in einem Düsenapparat. Dabei kann das dampfförmige TEDA im Gleich- oder im Gegenstrom mit dem flüssigen Lösungsmittel geführt werden. Vorteilhaft ist die Einleitung des dampfförmigen TEDA von oben in den Quenchapparat. Weiterhin vorteilhaft ist die tangentiale Zufuhr des flüssigen Lösungsmittels am Kopf des Fallfilmkondensators oder die Zufuhr des flüssigen Lösungsmittels durch eine oder mehrere Düsen, um eine vollständige Benetzung der Innenwand des Quenchapparates zu erreichen.

Die Menge des verwendeten Lösungsmittels wird nach Zweckmäßigkeitsgesichtspunkten ausgewählt. Im allgemeinen wird so verfahren, daß, je nach Art des Lösungsmittels, Lösungen mit einem TEDA-Gehalt von ca. 1 bis 50 Gew.-%, vorzugsweise 20 bis 40 Gew.-%, erhalten werden.

Im allgemeinen wird die Temperatur im TEDA-Quench durch Temperieren des eingesetzten Lösungsmittels und/oder des Quenchapparates auf 20 bis 100°C, vorzugsweise 30 bis 60°C, eingestellt.

Der Absolutdruck im TEDA-Quench beträgt im allgemeinen 0,5 bis 1,5 bar.

Wird bei der Reinigung des TEDA dieses entsprechend der DE 101 00 943.7 aus einer Mischung mit einem Lösungs- oder Verdünnungsmittel verdampft, weist das Lösungs- oder Verdünnungsmittel vorzugsweise bei Normaldruck einen Siedepunkt von 180 bis 250°C, insbesondere von 180 bis 230°C, insbesondere von 190 bis 210°C auf.

Als Lösungs- oder Verdünnungsmittel, das die Mischung enthält, aus der das TEDA verdampft wird, eignen sich besonders inerte
- polare aprotische Lösungsmittel beispielsweise Alkyl-2-pyrrolidone, beispielsweise N-Methyl-2-pyrrolidon (NMP), 1-Ethyl-2-pyrrolidon, 1,5-Dimethyl-2-pyrrolidon, 1-Isopropyl-2-pyrrolidon, Ether, beispielsweise Diethylenglykoldiethylether, Triethylenglykoldimethylether und Triethylenglykoldiethylether, Ketone, beispielsweise Acetophenon und Propiophenon, Lactone, beispielsweise γ-Butyrolacton, Sulfoxide, beispielsweise Dimethylsulfoxid, Carbonsäureester, beispielsweise Fumarsäuredimethylester, Nitrile, beispielsweise Benzonitril, und Harnstoffe, beispielsweise 1,3-Dimethyl-imidazolidin-2-on (DMEU) und Tetramethylharnstoff,
- cyclische oder acyclische Kohlenwasserstoffe, insbesondere gesättigte cyclische oder acyclische Kohlenwasserstoffe, beispielsweise Undecan, Dodecan, cis-Dekalin und trans-Decalin,
- chlorierte aliphatische Kohlenwasserstoffe beispielsweise 1-Chloroctan und 1,1-Dichloroctan,
- aromatische Kohlenwasserstoffe, Nitroaromaten und Phenole, beispielsweise Naphthalin, n-Butylbenzol, Phenol, Kresol, Nitrobenzol und Nitrophenol,
- chlorierte aromatische Kohlenwasserstoffe, beispielsweise 1,2-Dichlorbenzol, Benzylchlorid, 1,2,3,4-Tetramethylbenzol und 1,2,3,5-Tetramethylbenzol,
- Alkohole, beispielsweise Benzylalkohol, 2-Ethylhexanol, 1-Octanol, i-Decanol, 1,2-Propandiol, 1,3-Propandiol, Ethylenglykol, Diethylenglykol, 1,2-Propylenglykol, 1,3-Propylenglykol, 1,2-Butandiol, 1,3-Butandiol, 2,3-Butandiol, 1,4-Butandiol, Neopentylglykol, Diethylenglykolmonomethylether und Dipropylenglykol,
- primäre, sekundäre und tertiäre Amine, beispielsweise Tri-n-Butylamin, Benzylamin, Anilin, N-Ethylanilin, N,N-Dimethylanilin und N-N-Diethylanilin,
- N-Alkylamide, beispielsweise N-Methylformamid und N-Methylacetamid und deren Gemische.

Besonders bevorzugt sind polare aprotische Lösungs- oder Verdünnungsmittel mit einem E^{N}_{T}-Wert von 0,1 bis 0,6, besonders von 0,2 bis 0,5, insbesondere von 0,3 bis 0,45.

(Zur Definition des E^{N}_{T}-Wertes siehe Ch. Reichardt, Solvents and solvent effects in organic chemistry, 2. Auflage, VCH 1988).

Ganz besonders bevorzugte Lösungsmittel sind NMP und Ethylenglykol.

Das Lösungs- oder Verdünnungsmittel, das die Mischung enthält, aus der das TEDA verdampft wird, wird bevorzugt nach der Synthese des TEDA dem rohen oder dem noch verunreinigten TEDA zugesetzt.

Das Lösungs- oder Verdünnungsmittel kann im einmaligen Durchlauf oder nach der Abtrennung der Schwersieder als Kreislauflösung eingesetzt werden.

Die Menge des verwendeten Lösungs- oder Verdünnungsmittel wird nach Zweckmäßigkeitsgesichtspunkten ausgewählt. Im allgemeinen wird so verfahren, daß je nach Art des Lösungs- oder Verdünnungsmittels Lösungen oder Mischungen mit einem TEDA-Gehalt von ca. 1 bis 90 Gew.-%, bevorzugt 40 bis 70 Gew.-%, erhalten werden.

Das Verdampfen des TEDA, optionsweise aus einer Mischung von diesem mit einem Lösungs- oder Verdünnungsmittel kann nach den dem Fachmann geläufigen Verfahren und Bedingungen erfolgen, z.B. in einer Destillations- oder Rektifikationsapparatur, wobei das TEDA gegebenenfalls zusammen mit dem Lösungs- oder Verdünnungsmittel vorgelegt wird.

Bevorzugt wird das dampfförmige TEDA am Kopf oder in einem Seitenabzug einer Destillationskolonne erhalten. Das dampfförmige TEDA im erfindungsgemäßen Verfahren besitzt im allgemeinen eine Reinheit von größer 90 Gew.-%, bevorzugt größer 95 Gew.-%, insbesondere größer 97 Gew.-%.

Wenn eine Mischung enthaltend TEDA und das Lösungs- oder Verdünnungsmittel, aus der das TEDA erfindungsgemäß verdampft wird (z.B. des Sumpfes der entsprechenden TEDA-Destillationskolonne), eingesetzt wird, wird die Temperatur der Mischung durch Wahl des einzusetzenden Lösungs- oder Verdünnungsmittels, des TEDA-Gehalts der Mischung und/oder des Drucks, auf ≤ 230°C, bevorzugt 190 bis 210°C eingestellt. Der Absolutdruck beträgt hierbei im allgemeinen 0,1 bis 5 bar, bevorzugt 0,5 bis 1,5 bar.

Die Zeitdauer zwischen Anfall des im erfindungsgemäßen Verfahren verwendeten dampfförmigen TEDAs und TEDA-Quench beträgt vorteilhafterweise ≤ 10 Sekunden.

Das zu reinigende TEDA kann nach bekannten Verfahren, z.B. durch Umsetzung von Monoethanolamin, Diethanolamin, Triethanolamin, Ethylendiamin, Diethylentriamin, Triethylentetramin, Piperazin, N-(2-Hydroxyethyl)-piperazin, N,N'-Bis(2-Hydroxyethyl)-piperazin, N-(2-Aminoethyl)-piperazin, N,N'-Bis(2-Aminoethyl)-piperazin, Morpholin oder Mischungen hiervon an einem Katalysator, beispielsweise Metallpyrophosphate, Metallphosphate, beispielsweise Erdalkalimonohydrogenphosphat, Zeolithe, Zirkoniumphosphate, Al₂O₃, SiO₂, phosphorhaltiges TiO₂ oder ZrO₂ bei erhöhter Temperatur, im allgemeinen 250 bis 450°C, erhalten werden. Üblicherweise beträgt hierbei der Druck 0,1 bis 50, insbesondere 0,1 bis 5 bar. Optional kann die Umsetzung in Gegenwart eines inerten polaren aprotischen Lösungsmittels, wie N-Alkylpyrrolidonen, beispielsweise N-Methylpyrrolidon, Dioxan, THF, Dialkylformamiden, beispielsweise Dimethylformamid, Dialkylacetamiden, beispielsweise Dimethylacetamid, oder eines inerten polaren protischen Lösungsmittels, beispielsweise Wasser, und eines inerten Trägergases, beispielsweise N₂ oder Ar, durchgeführt werden.

Anschließend wird das TEDA aus der Mutterlauge auskristallisiert und durch Fest-Flüssig-Trennung abgetrennt. Die so erhaltene Mutterlauge wird gemäß der vorliegenden Erfindung in einer Extraktionsstufe intensiv mit einem Extraktionsmittel in Kontakt gebracht. Dabei findet ein Stoffaustausch statt. Das Extraktionsmittel wird so gewählt, daß das TEDA von diesem aufgenommen wird. Die für die Qualitätsminderung des TEDA verantwortlichen Neben- und Zersetzungsprodukte werden ebenfalls im Extraktionsmittel gelöst. Dadurch wird die Mutterlauge von diesen Neben- und Zersetzungsprodukten befreit und kann in den Prozeß rückgeführt werden.

Nach erfolgtem Stoffaustausch werden die an TEDA, Neben- und Zersetzungsprodukten verarmte Mutterlauge (Raffinatphase) und das an TEDA angereicherte Extraktionsmittel (Extraktphase) getrennt.

Das als Extraktionsmittel eingesetzte Lösungsmittel sollte vorteilhafter Weise die nachfolgenden Eigenschaften aufweisen:

Das Extraktionsmittel sollte mit dem Lösungsmittel, das in der Kristallisationsstufe eingesetzt wird, eine große Mischungslücke bilden, wobei die gegenseitige Löslichkeit von Extraktions- und Lösungsmittel < 10 Gew.-%, vorzugsweise < 1 Gew.-%, sein sollte. TEDA sollte sich in dem Extraktionsmittel wesentlich besser lösen als in dem Lösungsmittel, das in der Kristallisationsstufe eingesetzt wird. Weiterhin sollte das Extraktionsmittel gegenüber dem Lösungsmittel, das in der Kristallisationsstufe eingesetzt wird, einen ausreichenden Dichteunterschied aufweisen. Dadurch wird eine Trennung der Extrakt- und der Raffinat-Phase erleichtert. Der Dichtunterschied sollte vorzugsweise > 50 kg/m³, insbesondere > 100 kg/m³ sein.

Die erfindungsgemäß bevorzugt als Extraktionsmittel eingesetzten Lösungsmittel sind Wasser und mit Wasser mischbare Lösungsmittel wie niedere Alkohole oder zwei- oder mehrwertige Alkohole. Beispiele für geeignete Alkohole umfassen Methanol, Ethanol, n-Propanol, i-Propanol, Ethylenglykol, Polyethylenglykol und Glycerin. Die Alkohole können einzeln oder als Gemisch eingesetzt werden, gegebenenfalls auch im Gemisch mit Wasser. Das meistbevorzugte Extraktionsmittel ist Wasser.

Da bei der Extraktion neben dem TEDA auch die bei dessen Synthese und Reinigung anfallende Neben- und Zersetzungsprodukte in die Extraktphase übergehen, wird auf diese Art und Weise eine Mutterlauge (Raffinat-Phase) erhalten, die frei von TEDA und den unerwünschten Nebenprodukten ist. Die Mutterlauge kann rückgeführt und erneut als organisches Lösungsmittel, in das dampfförmiges TEDA eingeleitet und anschließend auskristallisiert wird, verwendet werden. Mit einer nicht erfindungsgemäß aufgearbeiteten Mutterlauge ist ein derartiges Rückfühten nicht möglich, da durch die in der Mutterlauge vorhandenen Verunreinigungen die Qualität des erhaltenen TEDA vermindert wird.

Die erfindungsgemäße Aufarbeitung der Mutterlauge mit einem Extraktionsmittel kann nach und unter den dem Fachmann geläufigen Verfahren und Bedingungen erfolgen, z.B. in einer Extraktionsapparatur, wobei die Mutterlauge jeweils zusammen mit dem Extraktionsmittel kontinuierlich oder diskontinuierlich vorgelegt wird.

Durch die erfindungsgemäße Extraktion wird die Menge des in der Mutterlauge enthaltenen TEDA auf Werte von < 10 Gew.-%, vorzugsweise < 1 Gew.-%, des ursprünglichen Werts vermindert. Entsprechend enthält die Extraktionsphase nach der erfindungsgemäßen Extraktion > 90 Gew.-%, vorzugsweise > 99 Gew.-%, des ursprünglich in der Mutterlauge enthaltenen TEDA. Je nach Art des eingesetzten Extraktionsmittels werden dabei Lösungen mit einem TEDA-Gehalt von ca. 1 bis 40 Gew.-%, vorzugsweise 5 bis 30 Gew.-%, insbesondere 10 - 30 Gew.-% erhalten.

Auch die Extraktionsphase kann gegebenenfalls nach Durchführen der erfindungsgemäßen Extraktion rückgeführt und erneut zur Extraktion eingesetzt werden. Es hängt von dem Sättigungsgrad an TEDA bzw. Neben- und Zwischenprodukten in der Extraktionsphase ab, ob bzw. wie oft die Extraktionsphase zurückgeführt werden kann. Der Sättigungsgrad hängt dabei selbstverständlich stark von der Natur des als Extraktionsmittels verwendeten

Lösungsmittels bzw. der Löslichkeit des TEDA und der Neben- bzw. Zwischenprodukte in diesem ab. Das Extraktionsmittel kann aber auch nach einmaliger Extraktion dem Prozess entnommen und aufgearbeitet werden. Dies wird insbesondere bei Verwendung von Wasser aufgrund dessen leichter Verfügbarkeit durchgeführt.

Ist ein gewisser Sättigungsgrad erreicht, so kann das zur Extraktion verwendete Lösungsmittel kein TEDA bzw. Nebenprodukt mehr aufnehmen und kann nicht mehr rückgeführt werden. Diese Extraktionsphase wird dann aufgearbeitet und in das verwendete Lösungsmittel und TEDA getrennt, vorzugsweise durch Destillation. Das Lösungsmittel wird dann erneut als Extraktionsmittel verwendet. Je nach Art und Durchführung der Aufarbeitung wird ein TEDA unterschiedlicher Reinheit erhalten. Im allgemeinen wird dieses dann in das Reinigungsverfahren rückgeführt, also verdampft, gegebenenfalls aus einer Mischung mit einem Lösungs- oder Verdünnungsmittel, und durch Einleiten in ein organisches Lösungsmittel kondensiert und aus diesem auskristallisiert. Das beschriebene Reinigungsverfahren kann auch direkt bei der Aufarbeitung der Extraktphase durch Destillation des aus der Kolonne austretenden TEDA durchgeführt werden.

Das erfindungsgemäße Verfahren erlaubt die Rückführung des in der Kristallisation des TEDA eingesetzten Lösungsmittels und somit eine Reduzierung der notwendigen Menge an frischem Lösungsmittel. Durch die Aufarbeitung der Extraktphase und die damit ermöglichte Rückführung des TEDA wird gegenüber der bisherigen Durchführung des Verfahrens auch die Ausbeute an TEDA erhöht.

Gemäß einer bevorzugten Ausführungsform läßt sich das erfindungsgemäße Verfahren wie folgt ausführen:

Eine Mischung enthaltend TEDA, die z.B. als Reaktionsaustrag in einem kontinuierlichen Verfahren durch Umsetzung von Ethylendiamin und Piperazin in einem Gasphasenreaktor bei 320 bis 420°C und 0,5 bis 1,5 bar in Gegenart eines Lösungsmittels (beispielsweise Wasser), eines Trägergases (z.B. N₂ oder Ar) und eines Zeolithkatalysators erhalten wurde, wird in eine Destillationsapparatur mit einer Destillationskolonne mit ca. 15 theoretischen Stufen geleitet. Hier werden Leichtsieder (beispielsweise Ammoniak, Ethylamin, Wasser) bei einer Kopftemperatur von 95 bis 120°C und einem Druck von im allgemeinen 500 mbar bis 1,5 bar über Kopf abgetrennt. Der Sumpfablauf wird in eine weitere Destillationskolonne mit ca. 30 theoretischen Stufen gepumpt. Bei einer Kopftemperatur von 140 bis 160°C und einem Druck von 500 mbar bis 1,5 bar wird in dieser Kolonne Piperazin über Kopf abgetrennt und optional wieder zurück zum Synthesereaktor gefahren.

Der Sumpfablauf enthaltend TEDA und Schwersieder wird in eine weitere Destillationskolonne mit ca. 25 theoretischen Stufen gepumpt. Bei einem Druck von 500 mbar bis 1,5 bar werden in dieser Kolonne die Schwersieder über den Sumpfablauf ausgeschleust. Am Kopf der Kolonne wird TEDA mit einer Reinheit von > 95 Gew.-%, insbesondere > 97 Gew.-%, über einen Teilkondensator dampfförmig abgezogen und in einem Fallfilmkondensator in einem Lösungsmittel, vorzugsweise Pentan und/oder Cyclohexan, bei einer Temperatur von 30 bis 100°C, bevorzugt 30 bis 60°C, direkt schockartig abgekühlt und gleichzeitig gelöst (TEDA-Quench).

Nach dem TEDA-Quench wird TEDA aus der Lösung in einer Kristallisationsstufe durch Verdampfen des Lösungsmittels bei einer Temperatur von 10 bis 100°C, bevorzugt 20 bis 40°C, und bei einem Druck von 0,1 bis 5 bar, bevorzugt 0,5 bis 1,5 bar oder durch Abkühlen bei einer Temperatur von im allgemeinen -10 bis 40°C, bevorzugt 0 bis 10°C, auskristallisiert.

Die aus dem Kristaller abgezogene Suspension wird in einer Fest-Flüssig-Trennung, z.B. in einer Zentrifuge, in hochreines TEDA und Mutterlauge getrennt. Die Mutterlauge, die noch Reste des Wertprodukts enthält, wird nun in einer Extraktionsstufe, beispielsweise einem Mixer/Settler oder einer Extraktionskolonne, bei einer Temperatur von 10 bis 100°C, bevorzugt 20 bis 40°C, und bei einem Druck von 0,1 bis 5 bar, bevorzugt 0,5 bis 1,5 bar, intensiv mit einem Extraktionsmittel, vorzugsweise Wasser, in Kontakt gebracht.

Die nach dem Stoffaustausch und der Phasentrennung aus der Extraktionsstufe austretende Extraktphase, die den Hauptanteil des TEDAs und die unerwünschten Neben- und Zersetzungsprodukte die zu einer Qualitätsminderung des TEDAs führen enthält, wird zurück zum Reaktor oder in eine Destillation geleitet. Das nach Destillation erhaltene TEDA kann in die Reinigungsstufe rückgeführt werden. Die Raffinat-Phase, die nur noch Spuren des TEDA enthält, wird zurück in den TEDA-Quench geleitet.

Die Erfindung wird nun in den nachfolgenden Beispielen näher erläutert:

### Beispiele

### Beispiel 1 (Vergleichsbeispiel):

Die Versuche wurden in einem mit elektrischen Heizbändern beheizten 4 l (Katalysatorvolumen) Salzbadreaktor (Rohrbündel aus 7 Rohren, Innendurchmesser 21 mm, Länge 2 m) aus rostfreiem Stahl durchgeführt. Die Rohrleitungen für den Reaktorfeed, Reaktoraustrag und den Destillationsteil waren zum Teil als Doppelmantelrohre ausgeführt und ölbeheizt. Die Anlagenteile waren schutzbeheizt und wurden durch Einsatz verschiedener Heizkreisläufe an die jeweils erforderliche Temperatur individuell angepasst. Als Katalysator wurde ein Zeolith in Form von Strängen (Durchmesser ca. 2 mm, Länge ca. 30 mm) verwendet (Katalysatorschüttung).

Das Einsatzgut von 1300 g/h sowie 3 Nl/h (Nl = Normliter = auf Normalbedingungen umgerechnetes Volumen) Stickstoff wurden bei Normaldruck in den auf 350°C beheizten Salzbadreaktor geleitet (Katalysatorbelastung: 1 kg Einsatzgut pro 1 Kat. (Schüttvolumen) und pro h).

Das Einsatzgut hatte folgende Zusammensetzung (Angaben in Gew.-%):

| | |
|---|---|
| Ethylendiamin | 30 % |
| Piperazin | 20 % |
| Wasser | 50 %. |

Das dampfförmige Reaktionsprodukt wurde in einem Quench mit Kreislaufflüssigkeit, die aus zuvor erhaltenem flüssigem Reaktionsprodukt bestand (siehe unten), bei 80°C kondensiert (= Reaktionsauftrags-Quench).

Die Analyse des Kondensats ergab folgende Zusammensetzung (Angaben in Gew.-%):

| | |
|---|---|
| Ammoniak | 3 % |
| Piperazin | 17% |
| Triethylendiamin | 23 % |
| Wasser | 54 % |
| Rest | Schwersieder und andere Nebenprodukte. |

Die nicht kondensierten Anteile wurden nach einem Gas-Flüssig-Abscheider in die Destillationskolonne abgeleitet.

Ein Teil des flüssigen Reaktionsproduktes wurde gekühlt und als Flüssigkeitskreislauf (für den Reaktionsauftrags-Quench) verwendet, ein anderer Teil wurde kontinuierlich mittels einer Pumpe in eine Destillationskolonne gepumpt. Die Glaskolonne mit einem Durchmesser von 50 mm war mit 30 Glockenböden ausgerüstet. Das Rücklaufverhältnis betrug etwa 1:1.

Die Leichtsieder (Ammoniak, Ethylamin, Wasser) wurden bei Normaldruck und einer Kopftemperatur von 96°C am Kopf der Kolonne flüssig abgezogen.

Der Sumpfablauf der Destillationskolonne wurde bei 155°C kontinuierlich in eine nachfolgende Destillationskolonne gepumpt.

Die Glaskolonne mit einem Durchmesser von 50 mm war mit 60 Glockenböden ausgerüstet. Das Rücklaufverhältnis betrug etwa 10:1. Piperazin wurde bei Normaldruck und einer Kopftemperatur von 150°C am Kopf der Kolonne flüssig abgezogen und zum Reaktor zurückgefahren.

In den Sumpf der Kolonne wurden 200 g/h N-Methyl-2-pyrrolidon eingeleitet.

Die Analyse des Sumpfablaufs ergab folgende Zusammensetzung (Angaben in Gew.-%):

| | |
|---|---|
| Piperazin | 0,03 % |
| Triethylendamin | 53 % |
| N-Methyl-2-pyrrolidon | 43 % |
| Rest | Schwersieder und andere Nebenprodukte. |

Der Sumpfablauf der Destillationskolonne wurde bei 185°C kontinuierlich in die nachfolgende Destillationskolonne gepumpt. Die Glaskolonne mit einem Durchmesser von 50 mm war mit 50 Glockenböden ausgerüstet. Das Rücklaufverhältnis betrug etwa 8:1. Die Schwersieder wurden kontinuierlich bei 200°C über den Kolonnensumpf ausgeschleust, die Vorlauftemperatur des ölbeheizten Verdampfers betrug 230°C. Am Kopf der Kolonne wurde TEDA dampfförmig abgezogen und bei ca. 30°C im Lösungsmittel Pentan (Mischung aus 80 Gew.-% n-Pentan und 20 Gew.-% iso-Pentan) schockartig abgekühlt und gleichzeitig gelöst (= TEDA-Quench). Für den TEDA-Quench wurde ein Fallfilmkondensator (Rieselfilm- oder Fallstromkondensator) eingesetzt, bei dem dampfförmiges TEDA von oben eingeleitet wurde. Die Pentanzufuhr erfolgte tangential am Kopf des Fallfilmkondensators. Die resultierende Lösung hatte folgende Zusammensetzung (Angaben in Gew.-%):

| | |
|---|---|
| Pentan | 94 % |
| Piperazin | 0,01 % |
| Triethylendiamin | 5 % |
| Rest | Nebenprodukte. |

Nach der teilweisen Abtrennung von Pentan durch Verdampfungskristallisation bei 25°C und einem Druck von 570 mbar wurde kristallines TEDA in einer Filternutsche von der Mutterlauge abgetrennt. TEDA wurde in einer Reinheit von mindestens 99,5 Gew.-% erhalten.

Ein Teil der Mutterlauge wurde mit dem bei der Verdampfungskristallisation erhaltenen Pentan und 200 g/h Frisch-Pentan zurück zum TEDA-Quench geleitet. Um eine Aufkonzentrierung von unerwünschten Neben- und Zersetzungsprodukten, die zu einer Qualitätsminderung des TEDAs führen zu verhindern, wurden ca. 200 g/h Mutterlauge ausgeschleust.

Die Analyse der ausgeschleusten Mutterlauge ergab folgende Zusammensetzung (Angaben in Gew.-%):

| | |
|---|---|
| Pentan | 95 % |
| Piperazin | 0,02 % |
| Triethylendiamin | 4,7 % |
| Rest | Nebenprodukte. |

### Beispiel 2 (erfindungsgemäß):

Die Durchführung des Versuchs geschah wie in Beispiel 1 beschrieben, jedoch wurde die Mutterlauge mit Wasser im Verhältnis von etwa 1:1 gemischt, geschüttelt und nach der Phasentrennung analysiert.

Die Analyse der Extraktphase ergab folgende Zusammensetzung (Angaben in Gew.-%):

| | |
|---|---|
| Pentan | <0,01% |
| Piperazin | 0,02 % |
| Triethylendiamin | 4,4 % |
| Wasser | 95,5 % |
| Rest | Nebenprodukte. |

Die Analyse der Raffinat-Phase ergab folgende Zusammensetzung (Angaben in Gew.-%):

| | |
|---|---|
| Pentan | 99,95 % |
| Piperazin | < 0,001 % |
| Triethylendiamin | 0,02 % |
| Wasser | 0,02 % |
| Rest | Nebenprodukte. |

Die Extraktphase kann zurück in den Reaktor geleitet und die Raffinat-Phase kann zusammen mit dem bei der Verdampfungskristallisation erhaltenen Pentan und 50 g/h Frisch-Pentan zurück zum TEDA-Quench geleitet werden.

Durch die Rückführung kann die Frisch-Pentanmenge um ca. 75 % reduziert und der TEDA-Verlust infolge der Mutterlaugenausschleusung ebenfalls um ca. 75 % reduziert werden.

### Beispiel 3 (erfindungsgemäß):

Die Durchführung des Versuchs geschah wie in Beispiel 1 beschrieben, jedoch wurde die Mutterlauge mit Wasser im Verhältnis von etwa 1:4 gemischt, geschüttelt und nach der Phasentrennung analysiert.

Die Analyse der Extraktphase ergab folgende Zusammensetzung (Angaben in Gew.-%):

| | |
|---|---|
| Pentan | < 0,01 % |
| Piperazin | 0,2 % |
| Triethylendiamin | 23,6 % |
| Wasser | 71,7 % |
| Rest | Nebenprodukte. |

Die Analyse der Raffinatphase ergab folgende Zusammenstzung (Angaben in Gew.-%):

| | |
|---|---|
| Pentan | 99,92 % |
| Piperazin | > 0,001 % |
| Triethylendiamin | 0,04 % |
| Wasser | 0,01 % |
| Rest | Nebenprodukte. |

Die Extraktphase kann zurück in den Reaktor geleitet und die Raffinatphase kann zusammen mit dem bei der Verdampfungskristallisation erhaltenen Pentan und 50 g/h Frisch-Pentan zurück zum TEDA-Quench geleitet werden.

Durch die Rückführung kann die Frisch-Pentanmenge um ca. 75 % reduziert und der TEDA-Verlust infolge der Mutterlaugenausschleusung ebenfalls um ca. 75 % reduziert werden.

## Patentansprüche

1. Verfahren zur Reinigung von Triethylendiamin (TEDA), bei dem TEDA verdampft und das dampfförmige TEDA in ein flüssiges Lösungsmittel eingeleitet und anschließend aus diesem auskristallisiert wird, **dadurch gekennzeichnet, daß** die nach dem Auskristallisieren des TEDA erhaltene Mutterlauge mit einem Extraktionsmittel extrahiert wird, das nicht oder nur gering mit dem Lösungsmittel der Mutterlauge mischbar ist und in der sich TEDA gut löst, und die nach Extraktion erhaltene, an TEDA verarmte Mutterlauge und/oder das zur Extraktion verwendete, mit TEDA angereicherte Extraktionsmittel in den Prozeß zurückführt.

2. Verfahren nach Anspruch 1, **dadurch gekennzeichnet, daß** das TEDA vor dem Einleiten in das flüssige Lösungsmittel aus einer Mischung enthaltend ein Verdünnungs- oder Lösungsmittel, das bei Normaldruck einen Siedepunkt von 175 bis 250°C aufweist, verdampft wird.

3. Verfahren nach Anspruch 1 oder 2, **dadurch gekennzeichnet, daß** die gegenseitige Löslichkeit von Extraktions- und in der Kristallisationsstufe eingesetztem Lösungsmittel < 10 Gew.-%, vorzugsweise < 1 Gew.-% beträgt.

4. Verfahren nach einem der Ansprüche 1 bis 3, **dadurch gekennzeichnet, daß** der Dichteunterschied zwischen Extraktions- und in der Kristallisationsstufe verwendetem Lösungsmittel > 50 kg/m³, insbesondere > 100 kg/m³, ist.

5. Verfahren nach einem der Ansprüche 1 bis 4, **dadurch gekennzeichnet, daß** das Extraktionsmittel ausgewählt ist aus der Gruppe bestehend aus Wasser, mit Wasser mischbaren niederen Alkoholen und mit Wasser mischbaren zwei- und mehrwertigen Alkoholen, vorzugsweise Wasser, Methanol, Ethanol, n-Propanol, i-Propanol, Ethylenglykol, Polyethylenglykol, Glycerin und Mischungen davon, insbesondere Wasser.

6. Verfahren nach einem der Ansprüche 1 bis 5, **dadurch gekennzeichnet, daß** das in der Kristallisationsstufe verwendete Lösungsmittel ausgewählt ist aus der Gruppe bestehend aus cyclischen und acyclischen Kohlenwasserstoffen, chlorierten aliphatischen Kohlenwasserstoffen, aromatischen Kohlenwasserstoffen, Alkoholen, Ketonen, aliphatischen Carbonsäuren, aliphatischen Nitrilen und Estern, insbesondere Pentan und/oder Dipropylenglykol ist.

7. Verfahren nach einem der Ansprüche 1 bis 6, **dadurch gekennzeichnet, daß** die Extraktion so durchgeführt wird, daß die Menge des in der Mutterlauge enthaltenen TEDA auf Werte von < 10 Gew.-%, vorzugsweise < 1 Gew.-%, reduziert wird.

8. Verfahren nach einem der Ansprüche 1 bis 7, **dadurch gekennzeichnet, daß** das mit TEDA angereicherte Extraktionsmittel aufgearbeitet wird, vorzugsweise durch Destillation.

9. Verfahren nach Anspruch 8, **dadurch gekennzeichnet, daß** das nach erfolgter Aufarbeitung erhaltenen TEDA in die Reinigungsstufe zurückgeführt wird.

10. Verfahren nach einem der Ansprüche 1 bis 9, **dadurch gekennzeichnet, daß** die Extraktion der Mutterlauge kontinuierlich oder diskontinuierlich erfolgt, vorzugsweise in einer Extraktionsapparatur.

## Claims

1. A process for the purification of triethylenediamine (TEDA) in which TEDA is vaporized and the gaseous TEDA is passed into a liquid solvent and is subsequently crystallized from this, wherein the mother liquor obtained after the TEDA has been crystallized is extracted with an extractant which is immiscible or only slightly miscible with the solvent of the mother liquor and in which TEDA is readily soluble, and the TEDA-depleted mother liquor obtained after extraction and/or the TEDA-enriched extractant which has been used for the extraction are/is returned to the process.

2. A process as claimed in claim 1, wherein the TEDA is vaporized from a mixture comprising a diluent or solvent which has a boiling point at atmospheric pressure of from 175 to 250°C before being passed into the liquid solvent.

3. A process as claimed in claim 1 or 2, wherein the mutual solubility of the extractant and the solvent used in the crystallization step is < 10% by weight, preferably < 1% by weight.

4. A process as claimed in any of claims 1 to 3, wherein the density difference between the extractant and the solvent used in the crystallization step is > 50 kg/m³, in particular > 100 kg/m³.

5. A process as claimed in any of claims 1 to 4, wherein the extractant is selected from the group consisting of water, water-miscible lower alcohols and water-miscible dihydric and polyhydric alcohols, preferably water, methanol, ethanol, n-propanol, i-propanol, ethylene glycol, polyethylene glycol, glycerol and mixtures thereof, in particular water.

6. A process as claimed in any of claims 1 to 5, wherein the solvent used in the crystallization step is selected from the group consisting of cyclic and acyclic hydrocarbons, chlorinated aliphatic hydrocarbons, aromatic hydrocarbons, alcohols, ketones, aliphatic carboxylic acids, aliphatic nitriles and esters, in particular pentane and/or dipropylene glycol.

7. A process as claimed in any of claims 1 to 6, wherein the extraction is carried out so that the amount of TEDA present in the mother liquor is reduced to < 10% by weight, preferably < 1% by weight.

8. A process as claimed in any of claims 1 to 7, wherein the TEDA-enriched extractant is worked up, preferably by distillation.

9. A process as claimed in claim 8, wherein the TEDA obtained after the work-up is returned to the purification step.

10. A process as claimed in any of claims 1 to 9, wherein the extraction of the mother liquor is carried out continuously or batchwise, preferably in an extraction apparatus.

## Revendications

1. Procédé de purification de triéthylènediamine (TEDA), dans lequel la TEDA est évaporée et la TEDA sous forme vapeur est introduite dans un solvant liquide et ensuite est séparée dans celui-ci à l'état cristallin, **caractérisé en ce que** la lessive mère obtenue après la séparation de la TEDA à l'état cristallin est extraite par un agent d'extraction qui n'est pas miscible ou uniquement faiblement au solvant de la lessive mère et dans lequel la TEDA se dissout bien, et la lessive mère obtenue après extraction et appauvrie en TEDA et/ou l'agent d'extraction utilisé pour l'extraction, enrichi en TEDA, sont recyclés dans le processus.

2. Procédé suivant la revendication 1, **caractérisé en ce que** la TEDA est évaporée avant l'introduction dans le solvant liquide à base d'un mélange contenant un diluant ou solvant qui présente sous pression atmosphérique standardisée un point d'ébullition de 175 à 250°C.

3. Procédé suivant la revendication 1 ou 2, **caractérisé en ce que** la solubilité réciproque de l'agent d'extraction et du solvant mis en oeuvre dans l'étape de cristallisation est <10% en poids, de préférence <1% en poids.

4. Procédé suivant l'une des revendications 1 à 3, **caractérisé en ce que** la différence de densité entre l'agent d'extraction et le solvant utilisé dans l'étape de cristallisation est >50 kg/m³, en particulier >100 kg/m³.

5. Procédé suivant l'une des revendications 1 à 4, **caractérisé en ce que** l'agent d'extraction est choisi parmi le groupe constitué d'eau, d'alcools inférieurs miscibles à l'eau et de diols et polyols miscibles à l'eau, de préférence de l'eau, du méthanol, de l'éthanol, du n-propanol, du i-propanol, de l'éthylèneglycol, du polyéthylèneglycol, de la glycérine et leurs mélanges, en particulier de l'eau.

6. Procédé suivant l'une des revendications 1 à 5, **caractérisé en ce que** le solvant utilisé dans l'étape de cristallisation est choisi parmi le groupe constitué d'hydrocarbures cycliques et acycliques, d'hydrocarbures aliphatiques chlorés, d'hydrocarbures aromatiques, d'alcools, de cétones, d'acides carboxyliques aliphatiques, d'esters et nitriles aliphatiques, en particulier du pentane et/ou du dipropylèneglycol.

7. Procédé suivant l'une des revendications 1 à 6, **caractérisé en ce que** l'extraction est effectuée de façon que la quantité de la TEDA contenue dans la lessive mère soit réduite à des valeurs <10% en poids, de préférence <1% en poids.

8. Procédé suivant l'une des revendications 1 à 7, **caractérisé en ce que** l'agent d'extraction enrichi en TEDA est traité, de préférence par distillation.

9. Procédé suivant la revendication 8, **caractérisé en ce que** la TEDA obtenue après que le traitement a eu lieu est recyclée dans l'étape de purification.

10. Procédé suivant l'une des revendications 1 à 9, **caractérisé en ce que** l'extraction de la lessive mère a lieu de manière continue ou discontinue, de préférence dans un appareil d'extraction.
